Europäisches Patentamt

European Patent Office

Office européen des brevets

(11) Veröffentlichungsnummer: **0 578 151 B1**

(12) **EUROPÄISCHE PATENTSCHRIFT**

(45) Veröffentlichungstag der Patentschrift: **11.10.95**

(51) Int. Cl.[6]: **C09J 133/08**, C08F 20/30,
//(C09J133/08,133:14)

(21) Anmeldenummer: **93110603.3**

(22) Anmeldetag: **02.07.93**

(54) **Schmelzhaftkleber für Medical-Produkte.**

(30) Priorität: **08.07.92 DE 4222334**

(43) Veröffentlichungstag der Anmeldung:
**12.01.94 Patentblatt 94/02**

(45) Bekanntmachung des Hinweises auf die
Patenterteilung:
**11.10.95 Patentblatt 95/41**

(84) Benannte Vertragsstaaten:
**DE ES FR GB IT NL**

(56) Entgegenhaltungen:
**WO-A-93/09152**

(73) Patentinhaber: **Beiersdorf Aktiengesellschaft
Unnastrasse 48
D-20245 Hamburg (DE)**

(72) Erfinder: **Kummer, Andreas B. Dr.
Altwiedenthaler Strasse 49
D-21147 Hamburg (DE)**
Erfinder: **Guse, Günther, Dr.
Ulenlock 1 c
D-21077 Hamburg (DE)**
Erfinder: **Harder, Christian, Dr.
Bredkamp 66
D-22589 Hamburg (DE)**
Erfinder: **Borchert, Gunter
Schiffbeker Weg 59
D-22119 Hamburg (DE)**

**Beschreibung**

Haftkleber auf Polyacrylat-Basis mit einpolymerisierten Benzoinderivaten sind aus den DE-PS 24 43 414 und 27 43 979 bekannt.

Sie eignen sich unter anderem zur Herstellung von Medical-Produkten. Dabei werden die Haftkleber durch Polymerisation in organischen Lösungsmitteln hergestellt. Die so gewonnene Lösung wird auf geeignete Trägermaterialien gestrichen. Die Lösungsmittel werden in energie-intensiven Trocknern verdampft und in aufwendigen Spezialanlagen zurückgewonnen.

Zum Zeitpunkt der genannten Patentanmeldungen, 1974 bzw. 1977, bestand noch kein Interesse daran, diese Herstellungstechnik aufzugeben. Zwar wurde beobachtet, daß die Haftkleber eine gewisse thermische Stabilität besaßen und sich in einem eigens improvisierten Laborversuch von Hand auch als Schmelze unter minimalen Schergefällen verstreichen ließen, doch kam dem keine technische Bedeutung zu, da geeignete Beschichtungsanlagen und leistungsfähige UV-Strahler nicht erhältlich waren (DE-PS 24 43 414, Spalte 4, Zeilen 3-10 und DE-PS 27 43 979, Spalte 3, Zeilen 19-26).

In den Folgejahren sind dann einige Schmelzhaftkleber beschrieben worden; auch wurde vorgeschlagen, sie für Medical-Produkte einzusetzen.
Beispielsweise wird im US-Patent 4,879,178 ein Schmelzhaftkleber beschrieben, der durch Kombination und Wechselwirkung von zwei Polymeren unterschiedlicher Glasübergangstemperatur entstehen soll. Zur Herstellung dieser Polymeren - die untereinander unverträglich sein dürften - werden wenig gebräuchliche Methacrylsäureester mit mehreren Heteroatomen in der Alkoholkette benötigt, so daß eine störungsfreie und wirtschaftliche Produktion von selbstklebenden Artikeln nach diesem Verfahren kaum denkbar erscheint.

In einigen anderen Fällen wird vorgeschlagen, Schmelzhaftkleber nach der Beschichtung durch energiereiche Strahlung zu vernetzen. Derartige Produkte sind aber überwiegend nicht für medizinische, sondern lediglich für technische Anwendungen geeignet.

Im Falle der US-Patentschrift 4,052,527 ergibt sich das schon daraus, daß Acrylsäureester verwendet werden, die mit polychlorierten Aromaten substituiert sind und daher toxikologischen Bedenken unterliegen.

In US 4,906,421 werden mehrschichtige, gegebenenfalls gefüllte Selbstklebemassen auf Basis Polyacrylat oder Kautschuk beschrieben, die als Schmelze (co)extrudierbar und durch ES härtbar sein sollen.

Die DE-OS 38 17 452 befaßt sich mit Acrylat-Schmelzhaftklebern, die unter Verwendung von wenig gebräuchlichen Monomeren wie Tetrahydrofurfuryl(meth)acrylat hergestellt werden. Sie haben relativ niedere mittlere Molekularmassen - hier als K-Werte ausgedrückt -, die der Fachmann bei Selbstklebemassen nicht bevorzugt. Nach Abtrennen der Lösemittel sollen die viskosen Schmelzen mit Benzophenon oder dessen Derivaten vermischt, schließlich aufgetragen und mit UV-Licht bestrahlt werden. Wegen der erforderlichen hohen Vernetzungsdichte und der vergleichsweise geringen Wirksamkeit der Sensibilisatoren erfordern solche Schichten lange Bestrahlungszeiten bzw. unwirtschaftlich geringe Fertigungsgeschwindigkeiten.

Auch in der deutschen Patentanmeldung P 39 42 232 sind Selbstklebemassen mit zugemischten Photosensibilisatoren vorgeschlagen worden. Eine Vernetzung durch UV-Bestrahlung wird dort neben der Elektronenbestrahlung erwähnt.

Alle Verfahren, bei denen einem Polymeren nachträglich ein Photosensibilisator zugemischt werden muß, leiden aber unter spezifischen Nachteilen. Schon das bloße Mischen ist ein verfahrenstechnisch unerwünschter Schritt, der großen Aufwand an Zeit und Rührenergie erfordert. Außerdem können die Sensibilisatoren bzw. ihr nach der Bestrahlung noch verbliebener Anteil in nicht gewünschter Weise migrieren oder eluiert werden.

Daher werden verschiedentlich Systeme beschrieben, bei denen Photosensibilisatoren in Schmelzhaftkleber einpolymerisiert werden sollen. Diese Sensibilisatoren nach dem Stand der Technik sind häufig Derivate des Benzophenons, die in mehrstufiger, nicht eben wirtschaftlicher Synthese dargestellt werden müssen.

Beispielhaft seien hier die Benzophenonderivate nach DE-OS 24 11 169, nach EP-OS 0 346 734, nach DE-OS 38 36 968 und nach DE-OS 38 44 445 genannt. Auch in US 4,871,812 wird ein Benzophenonderivat erwähnt; der Schwerpunkt dieser Erfindung scheint allerdings in einer Wechselwirkung zwischen Polyacrylaten und Carbonylamido-Gruppen enthaltenden Polymeren zu liegen.

In keinem der vorgenannten Fälle ist es jedoch gelungen, Schmelzhaftkleber für Medical-Produkte herzustellen, die einfach und wirtschaftlich herstellbare, hochwirksame Photosensibilisatoren in einpolymerisierter Form enthalten und demgemäß durch kurzzeitige Bestrahlung in den gewünschten Vernetzungszustand überführt werden können. Somit gibt es nach dem Stand der Technik kein Verfahren, das die Herstellung bahnförmiger, mit Schmelzhaftklebern beschichteter Medical-Produkte mit hohen Fertigungsgeschwindigkeiten erlauben würde.

Aufgabe der Erfindung ist es, diesem Mangel abzuhelfen.

In überraschender Weise wurde nun gefunden, und dies ist die Lösung der Aufgabe, daß die Verwendung von Haftklebern auf Polyacrylatbasis mit einpolymerisierten Benzoinderivaten zur großtechnischen kontinuierlichen Schmelzbeschichtung von Medical-Produkten mit anschließender Vernetzung des Klebers durch UV-Bestrahlung möglich ist.

Zu den Bedingungen der großtechnischen kontinuierlichen Schmelzbeschichtung gehören insbesondere hohe Temperatur, lange Verweilzeit und hohe Scherbeanspruchung des Schmelzhaftklebers und auch die Kombination von zwei oder allen Bedingungen, insbesondere von Temperatur und Verweilzeit.

Die eingangs schon erwähnten hochwirksamen Benzoinderivate waren für die Vernetzung von Polymerisaten bestimmt, die in Lösung hergestellt und relativ schonend aufgetragen wurden. Es war nicht zu erwarten, daß diese sensiblen Verbindungen auch für den Gebrauch in Schmelzhaftklebern geeignet sein würden. Vielmehr mußte erwartet werden, daß sie unter den Bedingungen einer Schmelzbeschichtung, insbesondere während einer längeren Verweilzeit bei hohen Temperaturen und/oder bei hoher Scherbeanspruchung instabil sein würden. Der Fachmann wurde von derartigen Versuchen abgehalten.

Überraschenderweise wurde nun aber gefunden, und auch dies ist Gegenstand der Erfindung, daß insbesondere die Benzoinderivate nach DE-PS 24 43 414 und 27 43 979 auch zur Vernetzung von Schmelzhaftklebern vorzüglich geeignet sind. Es erwies sich erstmals als möglich, Medical-Produkte bzw. ihre bahnförmigen Vormaterialien großtechnisch kontinuierlich mit Polyacrylat-Schmelzhaftklebern zu beschichten und den Kleber durch Kurzzeitbestrahlung mit UV-Lampen zu vernetzen.

Durch Vergleichsversuche konnte gezeigt werden, daß der covalente Einbau der Sensibilisatoren zu einer drastischen Beschleunigung der Vernetzung führt: wird in ein bestimmtes Polymer der gleiche Sensibilisator in gleicher Menge einerseits einpolymerisiert, andererseits nur zugemischt, so verläuft im ersten Fall die Vernetzung etwa um den Faktor 10 schneller.

Es bereitet z.B. keine Schwierigkeiten, Materialbahnen von 1200 mm Breite mit Geschwindigkeiten bis 80 m/min zu beschichten, mithin eine Stundenleistung bis 5760 $m^2$ beschichteten Materials zu erreichen. Bei Einsatz von Hochleistungs-UV-Strahlern, z.B. mit 120 W/cm Bogenlänge, ist es auch ohne weiteres möglich, im gleichen Durchlauf die Vernetzung vorzunehmen. Bei den genannten Mengen wäre dazu beispielsweise ein Strahlungsfeld von 1330 mm Länge vorzusehen, welches in 1 Sekunde durchlaufen wird.

An derartige Leistungen war in den 70er Jahren selbstverständlich nicht zu denken, da weder geeignete Auftragswerke noch entsprechend leistungsfähige UV-Strahler zur Verfügung standen. In den Folgejahren wurde der Wunsch nach einem Fertigungsverfahren mit Schmelzhaftklebern auch aus Gründen des Arbeits- und Umweltschutzes immer dringlicher, da bei ihnen der Umgang mit großen Mengen organischer Lösungsmittel entfällt, somit auch die Notwendigkeit ihrer Rückgewinnung und Aufarbeitung.

Aufwendige energieverzehrende Trocknungsanlagen, wie sie zur Entfernung von Lösungsmitteln oder auch von Wasser (bei wasserbasierten Klebersystemen, etwa Emulsionspolymerisaten) notwendig waren, können gänzlich entfallen. Hinzu kommt der Vorteil, daß erfindungsgemäß kleine, relativ einfach gebaute UV-Bestrahlungsstationen ausreichen, um die sensibilisierten Schmelzhaftkleber bei hohen Bahngeschwindigkeiten und entsprechend kurzen Bestrahlungszeiten zu vernetzen. Solche UV-Stationen erfordern wesentlich weniger apparativen und sicherheitstechnischen Aufwand als etwa Elektronenbestrahlungsanlagen nach den Stand der Technik.

Die Basispolymeren für die erfindungsgemäßen Schmelzhaftkleber können nach den üblichen Verfahren der Block- oder Lösungspolymerisation hergestellt werden. Insbesondere können die in den DE-PS 24 43 414 und DE-PS 27 43 979 beschriebenen Polymeren verwendet werden.

Als Monomere eignen sich die Acryl- und Methacrylsäureester von $C_4$- bis $C_{12}$-Alkanolen, Maleinsäure- und Fumarsäure-mono- oder -di-ester von $C_4$- bis $C_{12}$-Alkanolen sowie die Vinylester von $C_2$- bis $C_{12}$-Fettsäuren.

Zu geringeren Anteilen (≤ 40 Gew.-%) können Acryl- und Methacrylsäureester von $C_1$- bis $C_2$-Alkanolen, Vinylaromaten (wie Styrol) oder Vinylheterocyclen (wie N-Vinylpyrrolidon) einpolymerisiert werden, jeweils einzeln oder im Gemisch.

Weiterhin lassen sich Comonomere mit reaktiven Gruppen in Anteilen bis zu etwa 10 Gew.-% einbauen, z.B. ungesättigte Säuren (Acryl-, Methacryl-, Malein-, Fumarsäure), ungesättigte Amide (Acryl- und Methacrylamid sowie deren N-substituierte Derivate), Anhydride ungesättigter Säuren (Maleinsäureanhydrid).

In allen Fällen werden olefinisch ungesättigte Derivate des Benzoins einpolymerisiert.

Die Anteile können vorzugsweise 0,01 bis 5 Gew.-%, insbesondere aber 0,1 bis 2,0 Gew.-% des Monomergemisches betragen.

Besonders geeignet sind die schon in den DE-PS 24 43 414 und 27 43 979 als Photoinitiatoren beschriebenen Verbindungen Benzoinacrylat oder Benzoinmethacrylat oder Acrylsäure- oder Methacrylsäure-((2-alkoxy-2-phenyl-2-benzoyl)-ethyl)-ester. Vorzugsweise enthält die Alkoxygruppe 1 bis 4 Kohlenstoffatome und ist insbesondere Methoxy oder Ethoxy.

Falls man die Basispolymeren durch Lösungspolymerisation herzustellen wünscht, können als Lösungsmittel aliphatische Kohlenwasserstoffe (wie n-Heptan), aliphatische Ketone (wie Aceton, Ethylmethylketon), Ester gesättigter Carbonsäuren (wie Ethylacetat), Siedegrenzenbenzine oder die Gemische dieser Stoffe verwendet werden. Diese Lösungsmittel dienen ausschließlich der Polymerisation und werden im Kreislauf geführt, indem man sie in geeigneten bekannten Apparaturen wie etwa Dünnschichtverdampfern oder Entgasungsextrudern abtrennt und erneut einsetzt. Die Selbstklebeeigenschaften der Schmelzhaftkleber können selbstverständlich durch die Auswahl der obengenannten Monomeren beeinflußt werden, darüberhinaus aber in besonderen Fällen auch durch Zusatz von klebrigmachenden Harzen. Hier eignen sich besonders Kolophoniumester (wie Staybelite Ester 10) und synthetische Kohlenwasserstoffharze (wie Escorez 1102F) in Mengen bis zu 30 Gew.-%.

Schließlich können den Schmelzhaftklebern in Fällen, wo erhöhte thermische Stabilitäten erwünscht sind, an sich bekannte Alterungsschutzmittel zugemischt werden, wie etwa sterisch gehinderte Derivate des Phenols (wie die Irganox-Typen von CIBA-GEIGY), insbesondere in kleinen Mengen, vorzugsweise von 0,01 bis 0,3 Gew.-%.

Der Auftrag der erfindungsgemäßen Schmelzhaftkleber auf bahnförmige Trägermaterialien (wie Gewebe, Vliese und Folien) erfolgt mit den üblichen Schmelzbeschichtungsanlagen wie Walzenauftragswerken, Schmelzrotationssiebdruck- oder auch einfachen Schlitzdüsenauftragswerken, wobei das Polymerisat beispielsweise Temperaturen von 60 bis 200°C, bevorzugt 120° - 180°C oder aber Temperaturen von 80 bis 160°C, insbesondere 100 bis 140°C aufweisen kann.

Als bevorzugt seien Verweilzeiten in der Schmelzbeschichtungsanlage von bis zu 16 h bei 120°C oder bis 12 h bei 140°C sowie bis 6 h bei 160°C genannt, die von den erfindungsgemäßen Schmelzhaftklebern ohne Qualitätseinbuße ertragen werden. Dies war nicht zu erwarten, da andere Kleber bereits bei deutlich geringerer Belastung erhebliche Gelbildung zeigen, was eine erfindungsgemäße Weiterverwendung ausschließt.

Der Schmelzauftrg kann erfindungsgemäß unter hoher Scherbeanspruchung erfolgen, wie sie bei der großindustriellen kontinuierlichen Beschichtung üblicherweise auftritt. Die Massen haben sich als überraschend stabil erwiesen. Die Schmelzbeschichtung kann vollflächig, z.B. mittels Düsenauftrag oder segmentiert, z.B. durch Siebdruck erfolgen.

Die Vernetzung der erfindungsgemäßen Schmelzhaftkleber erfolgt durch kurzzeitige UV-Bestrahlung mit handelsüblichen Quecksilber-Hochdruck- oder Mitteldrucklampen. Eine Inertgasabdeckung ist nicht erforderlich. Dagegen kann es angebracht sein, die Strahlerleistung der Bahngeschwindigkeit anzupassen oder die Bahn bei Langsamfahrt teilweise abzuschatten, um ihre thermische Belastung zu verringern. Die Bestrahlungszeit richtet sich nach Bauart und Leistung der jeweiligen Strahler; bei der Verwendung produktionsüblicher Geräte kann sie unter 1 Sekunde liegen. Es ist ohne weiteres möglich, auf diese Weise die für Medical-Artikel üblichen Vernetzungsgrade zu erreichen, z.B. 50 bis 70 Gew.-% toluol-unlösliche Anteile in der Klebemasse.

Gegenstand der Erfindung sind auch die nach der erfindungsgemäßen Verwendung von Haftklebern hergestellten bzw. erhaltenen oder erhältlichen selbstklebenden Medical-Produkte.

Gegenstand der Erfindung ist auch das Verfahren zur Herstellung von Medical-Produkten unter Verwendung von Haftklebern auf Polyacrylatbasis mit einpolymerisierten Benzoinderivaten zur großtechnischen kontinuierlichen Schmelzbeschichtung der Medical-Produkte mit anschließender Vernetzung des Klebers durch UV-Bestrahlung sowie das Verfahren zur Herstellung der erfindungsgemäßen Schmelzbeschichtung mit der beschriebenen Verwendung von Haftklebern.

Als Medical-Produkte werden allgemein Materialien bezeichnet, die auf medizinischem Gebiet verwendet werden. Bevorzugte selbstklebende Medical-Produkte sind Pflaster, Wundpflaster, Verbandmaterialien, Wundverbände, Binden, Klebestreifen für medizinische Zwecke und Fixierstreifen für medizinische Gegenstände.

Die erzeugten Medical-Produkte - Rollenpflaster, Wundschnellverbände und andere selbstklebend ausgerüstete Medical-Produkte - werden einer epicutanen Prüfung an größeren Kollektiven von hautgesunden, freiwilligen Probanden unterzogen. Dabei werden Testpflaster für 24 h appliziert und die drei Kenngrößen Haftvermögen, Hautverträglichkeit und rückstandsfreies Abziehen beobachtet. Die statistische Auswertung ergibt, daß Produkte mit den erfindungsgemäßen Schmelzhaftklebern sicher haften, keine nennenswerten Hautirritationen hervorrufen und rückstandsfrei entfernbar sind.

Alle Mengenangaben, Anteile und Prozentanteile sind, soweit nicht anders angegeben, auf das Gewicht und die Gesamtmenge an Monomeren bezogen.

Die folgenden Beispiele dienen zur Erläuterung der Erfindung.

Beispiele 1 bis 5

Die folgenden Monomergemische (Mengenangaben in Gew.-%) wurden in Lösung copolymerisiert. Die Polymerisationsansätze bestanden aus 60 Gew.-% der Monomergemische sowie 40 Gew.-% Lösungsmittel (Benzin 60/95 : Aceton im Massenverhältnis 2 : 1). Die Lösungen wurden in üblichen Reaktionsgefäßen aus Glas oder Stahl (mit Rückflußkühler, Rührer, Temperaturmeßeinheit und Gaseinleitungsrohr) zunächst durch Spülen mit Stickstoff von Sauerstoff befreit und dann zum Sieden erwärmt.

Durch Zusatz von 0,2 bis 0,3 Gew.-% eines für die radikalische Polymerisation üblichen Initiators, wie Dibenzoylperoxid, Dilauroylperoxid oder Azobisisobutyronitril wurde die Polymerisation ausgelöst.

Während der Polymerisationszeit von etwa 20 Stunden wurde je nach Viskosität ggf. mehrmals mit weiterem Lösungsmittel verdünnt, so daß die fertigen Polymerlösungen Feststoffgehalte von etwa 35 bis 55 Gew.-% aufwiesen.

Tabelle 1

| Zusammensetzung der Schmelzhaftkleber für Medical-Produkte | | | | | |
|---|---|---|---|---|---|
| Beispiel | 1 | 2 | 3 | 4 | 5 |
| Monomer [Gew.-%] | | | | | |
| Benzoinacrylat | 0,5 | 0,5 | 0,5 | 1,0 | 1,0 |
| Methylacrylat | | | | 20,0 | |
| Butylacrylat | 18,5 | 18,5 | | 19,5 | |
| tert.-Butylacrylat | | | | | 40,0 |
| 2-Ethylhexylacrylat | 71,2 | 78,2 | 89,5 | 44,5 | |
| Dodecylacrylat | | | | | 54,0 |
| Methylmethacrylat | | | | | 5,0 |
| Acrylsäure | 2,8 | 2,8 | | | |
| N-tert.-Butylacrylamid | | | 10,0 | | |
| N-Vinylpyrrolidon | | | | 15,0 | |
| Bis-(2-ethylhexyl)fumarat | 7,0 | | | | |
| Regler [Gew.-%] | | | | | |
| Isopropanol | | | 1,0 | | 6,0 |
| Initiator [Gew.-%] | | | | | |
| | BPO | BPO | AIBN | BPO | LPO |
| | 0,2 | 0,2 | 0,2 | 0,3 | 3x0,2 |
| Ausbeute [%] | 99,6 | 99,0 | 99,6 | 96,4 | 98,3 |
| K-Wert [ ] | 63,1 | 67,2 | 74,8 | 67,4 | 71,0 |
| AIBN : Azobisisobutyronitril  BPO : Dibenzoylperoxid  LPO : Dilauroylperoxid | | | | | |

Die folgenden Prüfmethoden wurden angewendet.

K-Wert:

bestimmt nach den üblichen Methoden der Kapillarviskosimetrie (in Ubbelohde-Viskosimetern). Gemessen wurden die Lösungen von 1 g Polymer in 100 ml Toluol bei 25° C.

Klebkraft:

es wurden 25mm breite Streifen des Prüfmaterials auf geschliffene und entfettete Platten aus rostfreiem Stahl geklebt und fünfmal mit einer belasteten Walze hin und her überrollt (2 kg/cm Bandbreite, Geschwindigkeit: 10 m/min). Der Prüfstreifen wurde in einer Zugprüfmaschine von der Stahlplatte abgezogen und die dabei gemessene Kraft in Newton/cm Bandbreite angegeben (Abzugswinkel: 180° C, Geschwindigkeit: 30 cm/min).

5

Scherstandzeit:

es wurde ein etwa 15 cm langer und 13 mm breiter Streifen auf eine geschliffene und entfettete Platte aus rostfreiem Stahl so aufgeklebt, daß eine Länge von 20 mm auflag. Der Streifen wurde durch zweimaliges langsames Hin- und Herrollen mit einer 2 kg schweren Rolle angedrückt, das freie Ende des Streifens dann bei senkrechter Aufhängung mit einem Gewicht von 1000 g belastet. Die Prüfung wurde bei den jeweils angegebenen Temperaturen ausgeführt und die Zeit bis zum Abfallen des Streifens in Minuten angegeben.

Gelwert:

Proben von etwa 0,15 g Polymer wurden, gegebenenfalls mit der Trägerfolie, in Beutel aus Polyäthylen-Vlies eingeschweißt und 24 Stunden mit Toluol extrahiert. Der unlösliche Anteil blieb im Beutel und wurde nach Trocknung zurückgewogen. Gewichtsverluste des Vlieses und der Trägerfolie wurden gegebenenfalls als Blindwert berücksichtigt.

Beispiel 3a

Es wurde wie in Beispiel 3 verfahren, nur an Stelle des Benzoinacrylates wurde Acrylsäure-((2-methoxy-2-phenyl-2-benzoyl)-ethyl)-ester verwendet. Bei der Schmelzbeschichtung trat keine Qualitätseinbuße auf.

Man erhält für Medical-Produkte hervorragend geeignete Selbstklebemassen mit gutem Haftvermögen und guter Hautverträglichkeit, die sich auch wieder rückstandsfrei ablösen lassen.

Beispiel 6

Die Copolymerlösungen gemäß Beispielen 1 bis 5 wurden dann im Vakuum von den flüchtigen Anteilen befreit. Die so erhaltenen Schmelzhaftkleber wurden mittels eines Schlitzdüsenauftragswerkes auf Polyethylenterephthalat-Folien beschichtet.

Für die Massen gemäß den Beispielen 1 und 2 trat bei diesem Auftrag mit Verweilzeiten von sogar 36 h bei einer Massetemperatur von 120°C keine Qualitätseinbuße auf. Für die Masse gemäß Beispiel 3 betrug die Verweilzeit 6 h und die Temperatur 160°C, für die Masse nach Beispiel 3a betrug die Verweilzeit 6 h und die Temperatur 150°, für die Masse nach Beispiel 4 betrug die Verweilzeit 16 h bei einer Temperatur von 120°C und bei der Masse nach Beispiel 5 12 h bei 140°C. In allen Fällen trat trotz der zusätzlichen Scherbeanspruchung keine Qualitätseinbuße auf.

Die UV-Bestrahlung erfolgte mit einem Quecksilber-Hochdruckbrenner (Typ Hanau Q1200, Anschlußleistung 0,9 kW, Abstand 20cm).

Die Klebkräfte, Scherstandzeiten und Gelwerte wurden nach unterschiedlichen Bestrahlungszeiten bestimmt.

Tabelle 2:

Ausprüfung der in den Beispielen 1 bis 5 beschriebenen Schmelzhaftkleber nach der Beschichtung auf Polyesterfolie (Klebkräfte, Scherstandzeiten, Gelwerte)

Beispiel 6

| Masse gemäß Beispiel | | 1 | 2 | 3 | 4 | 5 |
|---|---|---|---|---|---|---|
| Masseauftrag | [g/m²] | 48 | 52 | 52 | 26 | 48 |
| | | | | | | |
| UV-Bestrahlung | [s] | 0 | 0 | 0 | 0 | 0 |
| Klebkraft | [N/cm] | del. | del. | del. | 3,6 | del. |
| Scherstandzeit | [min] | 1 | 1 | 1 | 20 | 28 |
| Gelwert | [%] | 0 | 0 | 0 | 0 | 0 |
| | | | | | | |
| UV-Bestrahlung | [s] | 15 | 14 | 16 | 10 | 15 |
| Klebkraft | [N/cm] | 3,1 | 3,6 | 4,3 | 3,7 | 2,9 |
| Scherstandzeit | [min] | 12 | 65 | 370 | 1096 | 124 |
| Gelwert | [%] | 29 | 52 | 56 | n.b. | 44 |
| | | | | | | |
| UV-Bestrahlung | [s] | 30 | 30 | 32 | 20 | 30 |
| Klebkraft | [N/cm] | 3,0 | 3,0 | 3,8 | 3,6 | 2,4 |
| Scherstandzeit | [min] | 890 | 750 | 45 | >15000 | 297 |
| Gelwert | [%] | 49 | 69 | 71 | 65 | 61 |

----------------

del. : delaminiert

Beispiel 7

Der erfindungsgemäße Schmelzhaftkleber nach Beispiel 2 wurde mittels Schmelzsiebdruck zusätzlich auch auf einen luftdurchlässigen Vliesträger beschichtet. Die Verweilzeit im Schmelzbehälter betrug 24 h, die Temperatur 130°C.

Tabelle 4:

Ausprüfung nach der Beschichtung auf Vliesträger
(Klebkraft, Gelwert)

Beispiel 7

Masseauftrag [g/m²] 30

UV-Bestrahlung [s] 16

Klebkraft [N/cm] 1,0

Gelwert [%] 61

Beispiel 8

Der erfindungsgemäße Schmelzhaftkleber nach Beispiel 3 wurde mittels Düsenauftrag zusätzlich noch auf ein Acetatseide-Gewebe beschichtet. Die Verweilzeit, im Schmelzbehälter betrug 10 h, die Temperatur 150°C.

Tabelle 5:

Ausprüfung nach der Beschichtung auf Gewebeträger (Klebkraft, Gelwert)

Beispiel 7

Masseauftrag [g/m²] 68

UV-Bestrahlung [s] 16

Klebkraft [N/cm] 4,4

Gelwert [%] 45

UV-Bestrahlung [s] 32

Klebkraft [N/cm] 3,6

Gelwert [%] 65

Bei dem Auftrag der Schmelze gemäß den vorstehenden Beispielen war kein merklicher Masseabbau (Abfall des K-Wertes) festzustellen. Die Massen zeichnen sich durch hervorragende thermische Stabilität und Scherstabilität aus.

**Patentansprüche**

1. Verwendung von Haftklebern auf Polyacrylatbasis mit einpolymerisierten Benzoinderivaten zur großtechnischen kontinuierlichen Schmelzbeschichtung von Medical-Produkten mit anschließender Vernetzung des Klebers durch UV-Bestrahlung.

2. Verwendung von Haftklebern gemäß Anspruch 1, dadurch gekennzeichnet, daß die Schmelzbeschichtung bei hoher Temperatur oder mit langer Verweilzeit oder unter starker Scherbeanspruchung erfolgt.

3. Verwendung von Haftklebern gemäß Anspruch 1, dadurch gekennzeichnet, daß die Schmelzbeschichtung bei hoher Temperatur und langer Verweilzeit erfolgt.

4. Verwendung von Haftklebern gemäß Anspruch 1, dadurch gekennzeichnet, daß die Schmelzbeschichtung bei hoher Temperatur und mit langer Verweilzeit und unter starker Scherbeanspruchung erfolgt.

5. Verwendung von Haftklebern nach Anspruch 1, dadurch gekennzeichnet, daß die Schmelzbeschichtung vollflächig, z.B. mittels Düsenauftrag oder segmentiert, z.B. durch Siebdruck erfolgt.

6. Verwendung von Haftklebern nach Anspruch 1, dadurch gekennzeichnet, daß die Vernetzung nach erfolgter Beschichtung durch kurzzeitige UV-Bestrahlung erfolgt.

7. Verwendung von Haftklebern nach Anspruch 1, dadurch gekennzeichnet, daß die UV-Vernetzung zu Gelwerten, d.h. toluolunlöslichen Anteilen von 50 bis 70 Gew.-% der Klebemasse führt.

8. Verwendung von Haftklebern nach Anspruch 1, dadurch gekennzeichnet, daß kleine Zusätze eines thermischen Alterungsschutzmittels erfolgen.

9. Medical-Produkte, die unter Verwendung der Haftkleber nach Anspruch 1 - 8 erhältlich sind.

10. Verfahren zur Herstellung von Medical-Produkten gemäß Anspruch 1 - 9 unter Verwendung von Haftklebern auf Polyacrylatbasis mit einpolymerisierten Benzoinderivaten zur großtechnischen kontinuierlichen Schmelzbeschichtung der Medical-Produkte mit anschließender Vernetzung des Klebers durch UV-Bestrahlung.

**Claims**

1. Use of polyacrylate-based pressure-sensitive adhesives with copolymerized benzoin derivatives for continuous large-scale industrial melt coating of medical products with subsequent crosslinking of the adhesive by UV irradiation.

2. Use of pressure-sensitive adhesives according to Claim 1, characterized in that the melt coating is carried out at a high temperature or with a long residence time or under high shearing stress.

3. Use of pressure-sensitive adhesives according to Claim 1, characterized in that the melt coating is carried out at a high temperature with a long residence time.

4. Use of pressure-sensitive adhesives according to Claim 1, characterized in that the melt coating is carried out at a high temperature and with a long residence time and under high shearing stress.

5. Use of pressure-sensitive adhesives according to Claim 1, characterized in that the melt coating is carried out over the entire surface, for example by means of nozzle application, or in segments, for example by screen printing.

6. Use of pressure-sensitive adhesives according to Claim 1, characterized in that the crosslinking after coating has taken place is carried out by brief UV irradiation.

7. Use of pressure-sensitive adhesives according to Claim 1, characterized in that the UV crosslinking leads to gel values, that is to say toluene-insoluble contents, of 50 to 70% by weight of the adhesive composition.

8. Use of pressure-sensitive adhesives according to Claim 1, characterized in that small amounts of a thermal antiageing agent are added.

9. Medical products which are obtainable using the pressure-sensitive adhesives according to Claims 1 - 8.

10. Process for the production of medical products according to Claims 1 - 9 using polyacrylate-based pressure-sensitive adhesives with copolymerized benzoin derivatives for continuous large-scale industrial melt coating of the medical products with subsequent cross-linking of the adhesive by UV irradiation.

**Revendications**

1. Utilisation d'adhésifs sensibles à la pression à base de polyacrylates avec des dérivés incorporés par polymérisation de la benzoïne en vue du revêtement par fusion en continu à l'échelle industrielle de produits médicaux, avec une réticulation consécutive de l'adhésif par irradiation aux rayons ultraviolets.

2. Utilisation d'adhésifs sensibles à la pression selon la revendication 1, caractérisée en ce que le revêtement par fusion se fait à température élevée ou à l'aide d'un temps de séjour prolongé ou sous une sollicitation de cisaillement forte.

3. Utilisation d'adhésifs sensibles à la pression selon la revendication 1, caractérisée en ce que le revêtement par fusion se fait à température élevée et à l'aide d'un temps de séjour prolongé.

4. Utilisation d'adhésifs sensibles à la pression selon la revendication 1, caractérisée en ce que le revêtement par fusion se fait à température élevée et à l'aide d'un temps de séjour prolongé et sous une sollicitation de cisaillement forte.

5. Utilisation d'adhésifs sensibles à la pression selon la revendication 1, caractérisée en ce que le revêtement par fusion se fait sur toute la surface, par exemple à l'aide d'une application par buse, ou de manière segmentée, par exemple par sérigraphie.

6. Utilisation d'adhésifs sensibles à la pression selon la revendication 1, caractérisée en ce que la réticulation se fait, après achèvement du revêtement, par une irradiation de courte durée aux rayons ultraviolets.

7. Utilisation d'adhésifs sensibles à la pression selon la revendication 1, caractérisée en ce que la réticulation aux rayons ultraviolets conduit à des valeurs de gel, c'est-à-dire à des proportions solubles dans le toluène de 50 à 70 % en poids de la masse adhésive.

8. Utilisation d'adhésifs sensibles à la pression selon la revendication 1, caractérisée en ce que l'on procède à de petites additions d'un agent thermique de protection contre le vieillissement.

9. Produits médicaux, que l'on peut obtenir par l'utilisation des adhésifs sensibles à la pression selon les revendications 1 - 8.

10. Procédé de préparation de produits médicaux selon les revendications 1 - 9, par utilisation d'adhésifs sensibles à la pression à base de polyacrylates avec des dérivés incorporés par polymérisation de la benzoïne en vue du revêtement par fusion en continu à l'échelle industrielle de produits médicaux, avec une réticulation consécutive de l'adhésif par irradiation aux rayons ultraviolets.